# EUROPEAN PATENT SPECIFICATION

(11) **EP 1 072 584 B1**
(45) Date of publication and mention of the grant of the patent: **05.11.2003**
(21) Application number: 99907918.9
(22) Date of filing: 12.03.1999
(51) Int. Cl.: C07C 251/24, C07C 217/58, C07C 227/16, C07C 229/34, C07D 213/74, C07F 7/18, C08F 8/30, C08G 85/00

(54) **AMINE DERIVATIVE FIXED TO RESIN AND METHOD FOR SYNTHESIZING BETA-AMINOCARBONYL COMPOUND IN A SOLID PHASE**
AN HARZ GEBUNDENE AMINDERIVATE UND VERFAHREN ZUR HERSTELLUNG EINER BETA-AMINOCARBONYLVERBINDUNG IN DER FESTPHASE
DERIVE D'AMINE FIXE A UNE RESINE ET PROCEDE DE SYNTHESE DE COMPOSE BETA-AMINOCARBONYLE DANS UNE PHASE SOLIDE

(30) Priority: 13.03.1998 JP 8277798
(43) Date of publication of application: 31.01.2001
(73) Proprietor: JAPAN SCIENCE AND TECHNOLOGY CORPORATION, Kawaguchi-shi, Saitama 332-0012 (JP)
(72) Inventor: KOBAYASHI, Shu, Chiyoda-ku, Tokyo 101-0064 (JP); AOKI, Yoji, Ota-ku, Tokyo 145-0066 (JP)
(74) Representative: Calamita, Roberto
(86) International application number: PCT/JP99/01221
(87) International publication number: WO 99/046239

(56) References cited:
- KOBAYASHI S. ET AL.: SYNLETT, 1995, pages 233-234, XP000942186
- KOBAYASHI S. et al., "p-Benzyloxybenzylamine (BOBA) Resin. A New Polymer-Supported Amine Used in Solid-Phase Organic Synthesis", TETRAHEDRON LETTERS, October 1998, Vol. 39, No. 40, p. 7345-7348, XP004133677
- KATRITZKY A.R. et al., "Synthesis of Primary Amines via Nucleophilic Addition of Organometallic Reagents to Aldimines on Solid Support", TETRAHEDRON LETTERS, 1997, Vol. 38, No. 40, p. 7011-7014, XP004090406
- BOYD E.A. et al., "Multiple Solid Phase Synthesis of (RS)-1-Aminophosphinic Acids", TETRAHEDRON LETTERS, 1996, Vol. 37, No. 10, p. 1647-1650, XP004030016
- KOBAYASHI S. et al., "Polymer-Supported Silyl Enol Ethers. Synthesis ans Reactions with Imines for the Preparation of an Amino Alcohol Library", TETRAHEDRON LETTERS, 1996, Vol. 37, No. 16, p. 2809-2812, XP004029804
- DEEGAN T.L. et al., "Non-Acidic Cleavage of Wang-Derived Ethers from Solid Support: Utilization of a Mixed-Bed Scavenger for DDQ", TETRAHEDRON LETTERS, 1997, Vol. 38, No. 28, p. 4973-4976, XP004081272

## Description

The present invention relates to a resin-immobilized β-aminocarbonyl compound and a solid state synthesis method thereof.

For peptide synthesis, for example, solid state synthesis methods using resin carriers have been known conventionally. Such solid state synthesis methods are effective means for simultaneous synthesis of a group of numerous types of analogous compounds. It has been suggested that solid state synthesis methods are applicable to various chemical reactions.

Practically, however, reactions to which solid state reaction is applicable are limited, disadvantageously, compared with liquid phase reaction as the principal reaction for chemical synthesis.

Katritzky, A.R. *et al*, TETRAHEDRON LETTERS, 1997, 38(40), 7011-7014 describes the preparation of primary amines by deriving resin-immobilized aldimines from the condensation of amine-functionalized Rink polystyrene resin with aldehydes, and reacting with Grignard reagents, lithium reagents or LiBH₄.

In the circumstances of the related art concerning solid state synthesis methods, the inventors of the present application have investigated the enhancement of the effectiveness of solid state synthesis methods by efficiently progressing the most essential and important reaction for generating carbon-carbon bonds in organic synthesis in the solid phase.

The inventors of the application have found a new method for iminoaldol-type addition reaction with a catalyst, using as the starting material an imine compound recovered from the reaction of an amine compound with aldehydes. Therefore, it has increasingly been an important problem to establish a solid state synthesis method capable of practically enhancing the efficiency of the method.

It is therefore an object of the present invention to provide new technical means for realizing the iminoaldol-type addition reaction as described above by a solid state synthesis method; more specifically, it is an object of the invention to provide a method for synthesizing a β aminocarbonyl compound utilizing a solid state synthesis reaction.

The present invention therefore provides a resin-immobilized β-aminocarbonyl compound represented by the following formula in a first aspect: 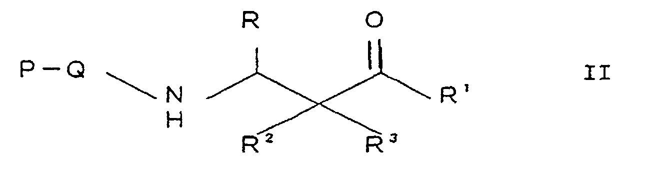 [ wherein P represents the principal chain of a resin polymer; Q represents a substituted or unsubstituted hydrocarbon side chain or a substituted or unsubstituted hydrocarbon side chain with a heteroatom interposed therein; R, and R² and R³ independently represent a substituted or unsubstituted hydrocarbon group or heterocyclic group; R¹ represents -OR⁰, -SR⁰ or R⁰ (R⁰ represents a substituted or unsubstituted hydrocarbon group or heterocyclic group)].

In a second aspect of -the invention, there is provided a resin-immobilized β-aminocarbonyl compound of the aforementioned formula [.wherein the hydrocarbon side chain Q represents the following formula:

-Q₁-O-Q₂-

(wherein Q₁ and Q₂ independently represent a substituted or unsubstituted hydrocarbon chain such as arylene, alkylenearylene or arylenealkylene)].

The invention provides a method for producing a resin-immobilized β-aminocarbonyl compound in a third aspect, comprising allowing a resin-immobilized imine of the formula I: P-Q-N=CH-R to react with silyl ether of the following formula: 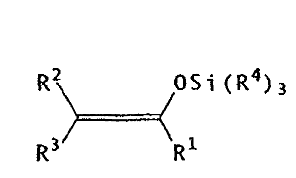 (wherein R⁴ represents a methyl group).

In a fourth aspect of the invention, there is provided a method for producing a β-aminocarbonyl compound represented by at least one of the following formulae (A) and (B), comprising releasing a resin-immobilized β-aminocarbonyl compound of the first and second aspects of the invention from the resin: 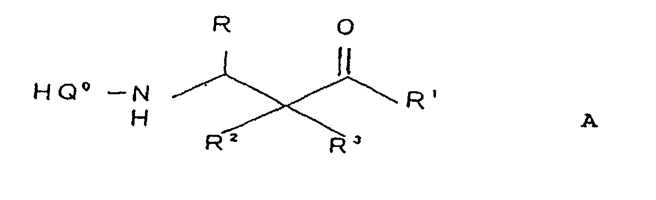 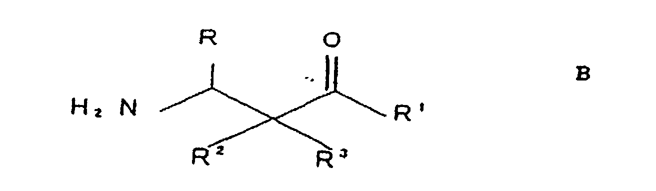 ( wherein Q⁰ represents a part of the side chain Q). In a fifth aspect of the invention, there in provided a method for producing β-aminocarbonyl compound, wherein the β-aminocarbonyl compound is released from the resin, by using Lewis acid.

The application provides a resin-immobilized β-aminocarbonyl compound represented by the formula II according to the first and second aspects of the invention. These have never been known to enable the solid state synthesis method of β-aminocarbonyl compound.

In the resin-immobilized β-aminocarbonyl compound of the formula II, as described above, P represents the principal chain of a resin polymer and Q represents a side chain to bind to the principal chain, wherein the resin polymer of the principal chain includes any of addition polymers, condensed polymers and cross-linked polymers thereof but preferably includes addition polymers of alkenes with carbon-carbon double bonds or cross-linked polymers thereof. The alkenes include aliphatic olefin and aliphatic diene and also include α, β-aliphatic unsaturated carboxylic acid or esters thereof, α, β-aliphatic unsaturated nitrile and aromatic alkenes such as styrene, α-methylstyrene and divinylbenzene. The addition polymers thereof or partially cross-linked polymers thereof are preferable.

It is needless to say that various condensed polymers of polyester, epoxy resins, polyether and polyamide are also included.

In accordance with the invention, the side chain Q includes substituted or unsubstituted hydrocarbon chains or substituted or unsubstituted hydrocarbon chains with heteroatoms such as oxygen atom or nitrogen atom interposed therein, wherein the hydrocarbon chains then include various hydrocarbon chains such as aliphatic, alicyclic, aromatic and aromatic aliphatic hydrocarbon chains and are for example alkylene chain represented by -(CH₂)ₙ- and phenylenealkylene chain. Otherwise, the hydrocarbon chains can satisfactorily be hydrocarbon chains with heteroatoms interposed therein.

These side chains Q are satisfactorily formed together with the principal chain P and are also satisfactorily formed by graft polymerization after the principal chain P is formed.

As the side chain Q, for example, hydrocarbon chains derived from the resin-immobilized amine P-Q₁-O-Q₂-NH₂ are provided in accordance with the invention, wherein Q₁ and Q₂ are arylene, alkylenearylene or arylenealkylene. Specifically, preferable examples of the hydrocarbon chains are hydrocarbon chains with Ph (phenylene chain) , such as -Ph-, -(CH₂)ₙ-Ph, -Ph-(CH₂)ₙ-, -Ph- (CH₂)ₙ-Ph-, particularly oxyphenylene chains, such as -Ph-O-Ph-, -Ph-(CH₂)ₙ-O-Ph-, -Ph-(CH₂)ₙ-O-(CH₂)ₙ-Ph.

These hydrocarbons can satisfactorily have various substituents with no inhibition of the solid state synthesis reaction but with an activity to activate the reaction. The substituents include hydrocarbon groups such as alkyl group and aryl group, halogen atom, alkoxyl group, acyloxy group, alkoxycarbonyl group, nitro group, cyano group and heterocyclic group.

R, R² and R³, and R⁰ composing R¹ in the resin-immobilized β-aminocarbonyl group of the formula II represent substituted or unsubstituted hydrocarbon groups or heterocyclic groups, wherein the hydrocarbon groups include various linear or cyclic aliphatic or aromatic or aromatic aliphatic hydrocarbon groups, saturated or unsaturated. Similarly, the heterocyclic groups include various heterocyclic groups containing oxygen or nitrogen. These are satisfactorily substituted with various substituents with no inhibition of the solid state synthesis reaction but with an activity to activate the reaction, for example hydrocarbon groups such as alkyl group and aryl group, halogen atom, alkoxyl group, acyloxy group, alkoxycarbonyl group, nitro group, cyano group and heterocyclic group.

In accordance with the invention, further, the resin-immobilized amine of the formula III P-Q₁-O-Q₂-NH₂ is provided as a substrate for the solid state synthesis of compounds containing nitrogen such as amino group. The resin-immobilized amine is essentially required for constructing a library of nitrogen-containing compounds for the solid state synthesis.

For example, -Q₁-O-Q₂- is more specifically described as such a structure as -Ph-CH₂-O-Ph-CH₂-.

The reaction of various resin-immobilized amines of formula III, including the preferred above-cited resin-immobilized amine with aldehydes generates the corresponding resin-immobilized imines of the formula I : P-Q-N=CH-R.

The resin-immobilized amine of the formula P-Q-NH₂ or P-Q₁-O-Q₂-NH₂, as produced by aminating for example a resin polymer of a structure P-QH and P-QX (in the formula, X represents halogen atom) or reducing an amide of the formula P-Q₁-O-Q₂-CONH₂ (in the formula, Q₁ represents a part of the side chain Q), is allowed to react with aldehydes.

The resin-immobilized imine synthesis through the reaction with aldehydes is appropriately carried out in a solvent at a temperature of about -10 to 70 °C, preferably about 10 to 60 °C. The solvent appropriately includes DMF, DMSO, nitriles and halogenated hydrocarbons.

During the reaction, aldehydes are appropriately used at an equivalent weight ratio to the resin-immobilized imine within a range of 1 to 10 during the reaction.

The resin-immobilized imine represented by the formula I can be used as a substrate for solid-phase iminoaldol reaction. The reaction is carried out by allowing the resin-immobilized imine of the formula I to react with the silyl ethers.

The solid state reaction with the silyl ethers is progressed in a solvent, using for example a rare earth Lewis acid catalyst. As the solvent, for example, use can be made of halogenated hydrocarbons, aromatic hydrocarbons, ethers, nitriles, alcohols and water or appropriate mixture solvents thereof.

The rare earth Lewis acid satisfactorily includes rare earth metal compounds with Lewis acidity, for example organic acid ester salts, alcoholates, organic metal compounds and organic complex compounds of rare earth elements such as ytterbium (Yb), scandium (Sc), yttrium (Y), lanthanoid (La), samarium (Sm) and neodium (Nd). Among them, rare earth triflate for example Yb(OTf)₃ is more appropriate.

The silyl ethers are used at an equivalent weight ratio of generally 0.5 to 10, preferably 1 to 7 to the resin-immobilized imine. The rare earth Lewis acid catalyst is used at an equivalent weight ratio of generally 0.01 to 1, preferably about 0.1 to 0.6.

The reaction temperature is appropriately -20 to 60 °C, more preferably ambient temperature or therearound.

In accordance with the invention, the resin-immobilized β-aminocarbonyl compound can be recovered by the solid state reaction.

Together with the amine and imine, the resin-immobilized β-aminocarbonyl compound serves as important means for constructing a library of various nitrogen-containing organic compounds.

By releasing the resin-immobilized β-aminocarbonyl compound from the immobilizing resin, the β-aminocarbonyl compound of at least one of the formulas A and B can be recovered.

As shown in the following examples, for example, the selectivity to the formula A or B depends on the conditions for the release reaction.

Lewis acid is effectively used for the release reaction. By using Lewis acid, the β-aminocarbonyl compound can be released under mild conditions in a smooth manner. Oxidative release is also effective.

The invention will now be described in more detail in the following examples.

### Example 1

According to the following reaction scheme, chloromethylated polystyrene 1 reacted with p-hydroxybenzamide in the presence of sodium hydroxide, followed by reduction with borane, to recover resin-immobilized amine 2.

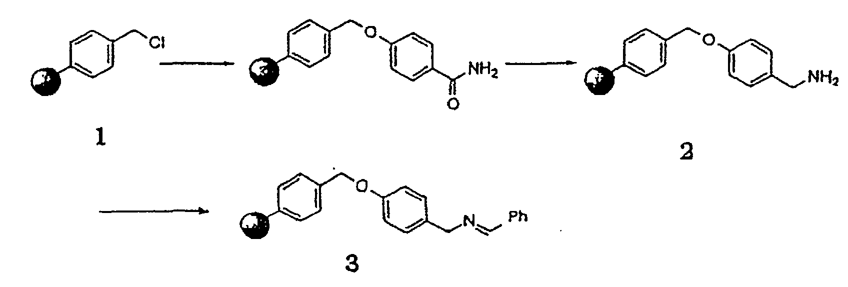

Benzaldehyde reacted with the resulting resin-immobilized amine 2, to recover the corresponding resin-immobilized imine 3.

According to the following reaction scheme, the resin-immobilized imine 3 reacted with silyl ether in the presence of a rare earth Lewis acid catalyst Yb(OTf)₃ of 20 mol % in a solvent dichloromethane at ambient temperature: 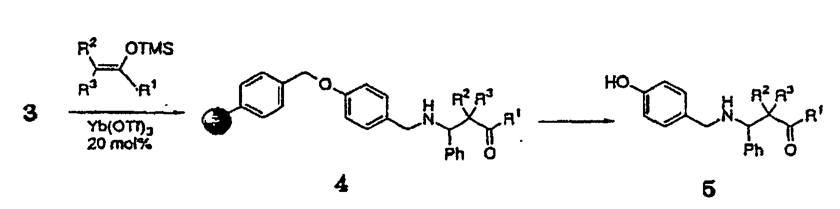 [in the formula, TMS represents trimethylsilyl group]. The resulting resin-immobilized β-aminocarbonyl compound 4 was subjected to a release process from the solid phase by using Lewis acid, to recover β-amino ester as β-aminocarbonyl compound 5, wherein the hydroxybenzyl group is bonded to the amino group.

The silyl ether type and release conditions were modified. The results are shown below in Table 1.

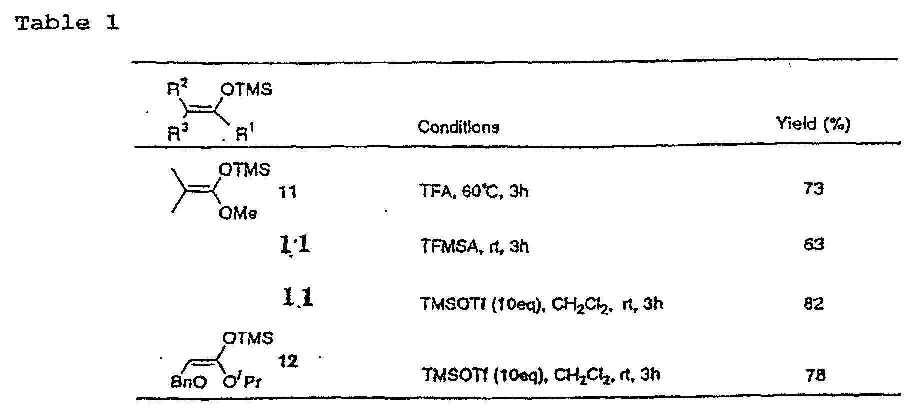

The physico-chemical properties are shown below. R = Ph, R², R3 = Me, R¹ = OMe

**Table 2**

| | |
|---|---|
| ¹ H | 1. 03 (3H, s), 1. 11 (3H, s), 3. 32 (1H, d, 13. 2Hz), 3. 57 (1H, d, 13. 2Hz), 3. 65 (3H, s), 3. 88 (1H, s), 6. 74 (2H, d, 8. 6Hz), 7. 06 (2H, d, 8. 6Hz), 7. 25-7. 36 (5H, m) |
| | |
| ¹³C | 19. 5, 24. 2, 47. 6, 50. 9, 52. 0, 67. 7, 115. 1, 127. 5, 128. 0, 129. 1, 129. 7, 132. 4, 139. 1, 154. 8, 178. 2 |
| | |
| IR | 3397, 1734cm⁻¹ |

### Example 2

The same procedures as in Example 1 were carried out, except for the use of the rare earth Lewis acid catalyst at 30 mol % for reaction of various resin-immobilized imines with silyl ether and except that the resulting product was released from the solid phase by using a Lewis acid TMSOTf.

The results are shown in Table 3.

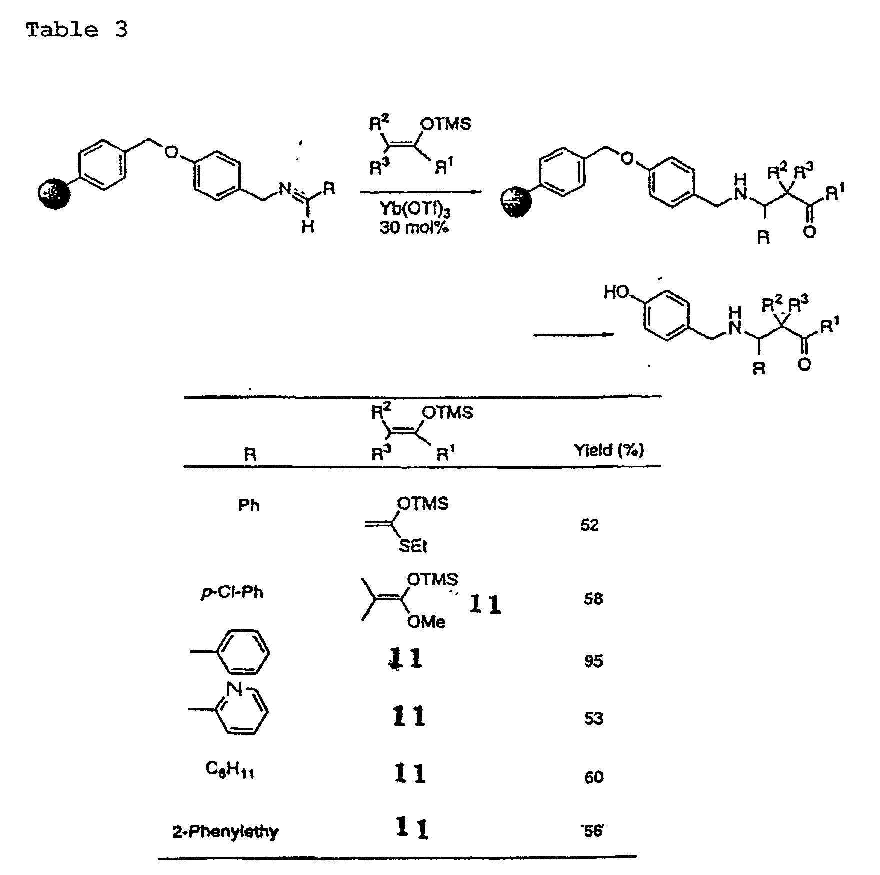

The physico-chemical properties are shown below.
R = Ph, R², R³ = H , R¹ = SEt

**Table 4**

| | |
|---|---|
| 1 H | 1. 21 (3H, t, 7. 5Hz), 2. 81-2. 99 (4H, m), 3. 45 (1H, d, 11. 7Hz), 3. 55 (1H, d, 11. 7 Hz), 4. 15 (1H, dd, 5. 0, 8. 6Hz), 6. 73 (2 H, d, 8. 4Hz), 7. 10 (2H, d, 8. 4Hz), 7. 19 -7. 40 (5H, m), |
| | |
| ¹³C | 12. 3, 21. 4, 47. 8, 51. 3, 58. 9, 115. 6, 127. 3, 128. 2, 128. 7, 130. 2, 131. 4, 141. 2, 198. 2 |
| | |
| R = P h ― C I , R² , R ³ = M e , R ¹ = O M e | |

**Table 5**

| | |
|---|---|
| ¹ H | 1. O2 (3H, s), 1. 09 (3H, s), 3. 29 (1H, d, 13. 2Hz), 3. 55 (1H, d, 13. 2Hz), 3. 64 (3 H, s), 3. 84 (1H, s), 6. 75 (2H, d, 8. 6Hz), 7. 05 (2H, d, 8. 6Hz), 7. 20 (2H, d, 8. 4Hz), 7. 31 (2H, d, 8. 4Hz) |
| | |
| ¹³C | 19. 5, 24. 0, 31. 0, 47. 3, 50. 7, 51. 9, 66. 9, 115. 0, 128. 1, 129. 5, 130. 4, 132. 2, 133. 1, 137. 6, 154. 6, 177. 6 |
| | |
| R = 2-pyridyl, R² , R ³ = Me, R ' = OMe | |

**Table 6**

| | |
|---|---|
| ¹ H | 1. 06 (3H, s), 1. 14 (3H, s), 3. 30 (1H, d, J=13. 2Hz), 3. 57 (1H, d, J=13. 2Hz), 3. 64 (3H, s), 3. 96 (1H, s), 6. 70 (2H, d, J=8. 6Hz), 7. 05 (2H, d, J=8. 6Hz), 7. 20 (2H, m), 7. 64 (1H, t, 7. 7Hz), 8. 60 (1H, d, J=4. 2Hz) |
| | |
| ¹³C | 19. 9, 23. 5, 47. 6, 51. 4, 51. 8, 67. 7, 115. 0, 122. 3, 124. 6, 129. 6, 135. 7, 149. 0, 154. 8, 177. 6 |
| | |
| R = cyclohexyl, R² , R ³ = M e , R' = OMe | |

**Table 7**

| | |
|---|---|
| ¹ H | 1. 14 (3H, s), 1. 18 (3H, s), 1. 25-1. 65 (10H, m), 2. 09 (1H, m), 2. 66 (1H, m), 3. 64 (3H, s), 3. 73 (1H, d, 12. 6Hz), 3. 88 (1H, d, 12. 6Hz), 6. 76 (2H, d, 8. 4Hz), 7. 18 (2H, d, 8. 4Hz) |
| | |
| R = phenylethyl, R², R³ = Me , R' = OMe | |

**Table 8**

| | |
|---|---|
| ¹ H | 1. 08 (3H, s), 1. 15 (3H, s), 1. 55 (2H, m, 2. 48-2. 60 (2H, m), 3. 63 (3H, s), 3. 71 (1H, d, 12. 3Hz), 3. 84 (1H, d, 12. 3Hz), 6, 70-6. 74 (3H, m), 7. 10-7. 28 (6H, m) |
| | |
| ¹³C | 21. 4, 21. 8, 29. 7, 32. 8, 47. 7, 51. 8, 54. 0, 63. 3, 115. 3, 126. 0, 126. 5, 128. 3, 128. 4, 129. 7, 132. 1, 165. 9, 175. 1 |
| | |
| IR | 3337, 1732 |

### Example 3

The same procedures as in Example 2 were carried out except for the use of DDQ for releasing the product from the solid phase. The results are shown in Table 9.

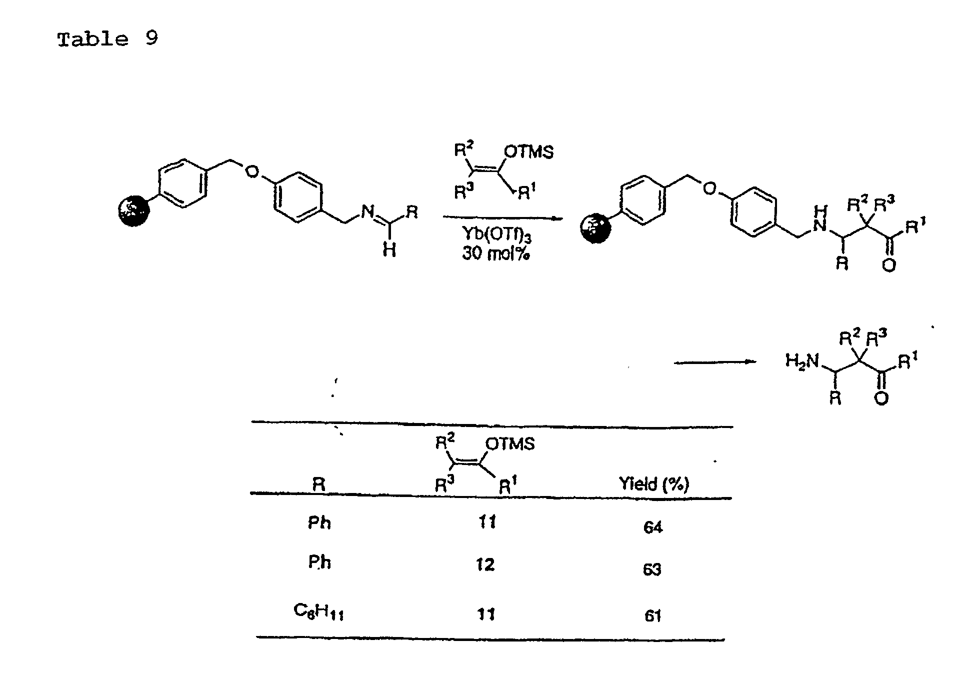

The physico-chemical properties are shown below.
R = P h , R ² , R ³ = M e , R¹ = OMe

**Table 10**

| | |
|---|---|
| ¹ H | 1. 09 (3H, s), 1. 15 (3H, s), 3. 70 (3H, s), 4. 24 (1H, s), 7. 28-7. 35 (5H, m) |
| | |
| ¹³C | 20. 6, 24. 2, 52. 0, 55. 7, 64. 5, 127. 4, 127. 8, 127. 9, 128. 3, 176. 9 |
| | |
| R = cyclohexyl, R ² , R ³ = M e , R' = OMe | |

**Table 11**

| | |
|---|---|
| ¹ H | 0. 99 (3H, s), 1. 22 (3H, s), 1. 46-1. 76 (10H, m), 1. 96 (1H, m), 2. 84 (1H, d, 6. 2 Hz), 3. 64 (3H, s) |
| | |
| ¹³C | 19. 5, 22. 9, 25. 4, 25. 7, 25. 9, 26. 2, 48. 4, 54. 5, 62. 0 |

As has been described above in detail, the solid state synthesis of β-aminocarbonyl compound through the application of iminoaldol reaction is attained in accordance with the invention of the application; and additionally, a library of β-aminocarbonyl compounds can efficiently be constructed.

## Claims

1. A resin -immobilized β-aminocarbonyl compound represented by the following formula: 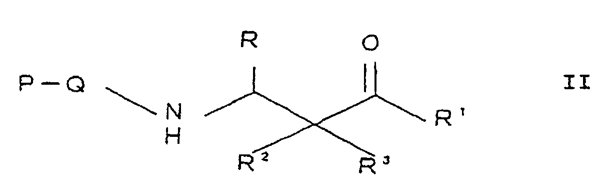 [:wherein P represents the principal chain of a resin polymer; Q represents a substituted or unsubstituted hydrocarbon side chain or a substituted or unsubstituted hydrocarbon side chain with a heteroatom interposed therein; R and R² and R³ independently represent a substituted or unsubstituted hydrocarbon group or heterocyclic group; R¹ represents -OR⁰, -SR⁰ or R⁰ (R⁰ represents a substituted or unsubstituted hydrocarbon group or heterocyclic group)].

2. A resin-immobilized β-aminocarbonyl compound as claimed in claim 1, wherein the hydrocarbon side chain Q represents the following formula:
-Q₁-O-Q₂-
(wherein Q₁ and Q₂ independently represent a substituted or unsubstituted hydrocarbon chain such as arylene, alkylenearylene or arylenealkylene).

3. A method for producing a resin-immobilized β-aminocarbonyl compound as claimed in claim 1 or claim 2, said method comprising allowing a resin-immobilized imine of the formula I: P-Q-N=CH-R to react with silyl ether of the following formula: 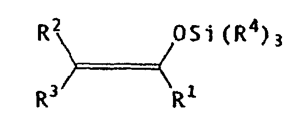 ( wherein R⁴ represents a methyl group).

4. A method for producing a β-aminocarbonyl compound represented by at least one of the following formulae (A) and (B), comprising releasing a resin-immobilized β-aminocarbonyl compound as claimed in claim 1 or claim 2 from the resin: 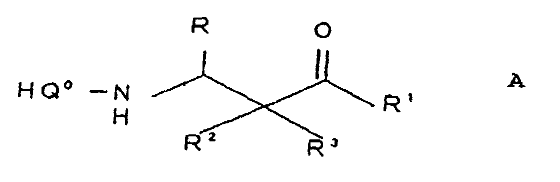 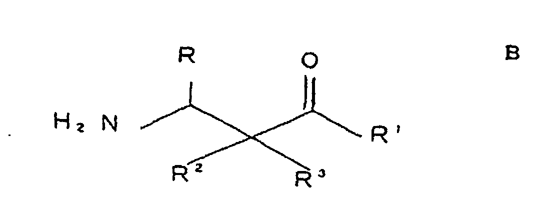 ( wherein Q⁰ represents a part of the side chain Q).

5. A method for producing aβ-aminocarbonyl compound as claimed in claim 4,wherein the β-aminocarbonyl compound is released from the resin, by using Lewis acid.

## Patentansprüche

1. Harz-immobilisierte β-Aminocarbonylverbindung der folgenden Formel: 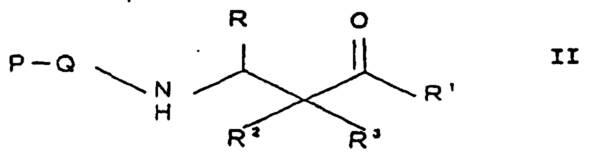 worin P für die Hauptkette eines Harzpolymers steht; Q für eine gegebenenfalls substituierte Kohlenwasserstoffseitenkette oder eine gegebenenfalls substituierte Kohlenwasserstoffseitenkette mit einem eingeschobenen Heteroatom steht; R, R² und R³ unabhängig voneinander für eine gegebenenfalls substituierte Kohlenwasserstoffgruppe oder eine heterozyklische Gruppe stehen; R¹ für -OR⁰, -SR⁰ oder R⁰ steht (R⁰ steht für eine gegebenenfalls substituierte Kohlenwasserstoffgruppe oder eine heterozyklische Gruppe).

2. Harz-immobilisierte β-Aminocarbonylverbindung nach Anspruch 1, wobei die Kohlenwasserstoffseitenkette Q für die folgende Formel steht:
- Q₁-O-Q₂₋ -
worin Q₁ und Q₂ unabhängig voneinander für eine gegebenenfalls substituierte Kohlenwasserstoffkette wie Arylen, Alkylenarylen oder Arylenalkylen stehen.

3. Verfahren zur Herstellung einer Harz-immobilisierten β-Aminocarbonylverbindung nach Anspruch 1 oder 2, bei dem man ein Harz-immobilisiertes Imin der Formel I: P-Q-N=CH-R mit einem Silylether der folgenden Formel umsetzt: 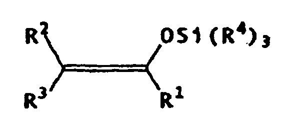 worin R⁴ für eine Methylgruppe steht.

4. Verfahren zur Herstellung einer β-Aminocarbonylverbindung wenigstens einer der folgenden Formeln (A) und (B), bei dem man eine Harz-immobilisierte β-Aminocarbonylverbindung nach Anspruch 1 oder 2 vom Harz freisetzt: 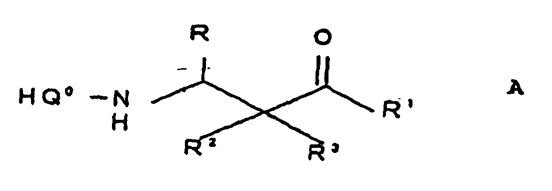 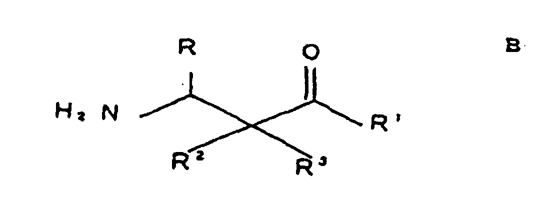 worin Q⁰ für einen Teil der Seitenkette Q steht.

5. Verfahren zur Herstellung einer β-Aminocarbonylverbindung nach Anspruch 4, bei dem man die β-Aminoarbonylverbindung mittels einer Lewis-Säure vom Harz freisetzt.

## Revendications

1. Composé β-aminocarbonylé immobilisé sur une résine représentée par la formule suivante: 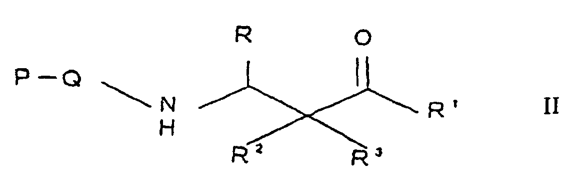 où P représente la chaîne principale polymère de la résine ; Q représente une chaîne latérale hydrocarbonée substituée ou non-substituée ou une chaîne latérale hydrocarbonée substituée ou non-substituée avec un hétéro atome intercalé; R, R² et R³ représentent indépendamment un groupe hydrocarboné ou hétérocyclique substitué ou non-substitué ; R¹ représente les groupements -OR⁰, -SR⁰ ou R⁰ (R⁰ représente un groupe hydrocarboné ou hétérocyclique substitué ou non-substitué).

2. Composé β-aminocarbonylé immobilisé sur une résine selon la revendication 1 où la chaîne latérale hydrocarbonée Q représente la formule suivante:
-Q₁-O-Q₂
(où Q₁ et Q₂ représentent indépendamment une chaîne hydrocarbonée substituée ou non-substituée telle que l'arylène, l'alkylènearylène ou arylènealkylène).

3. Procédé pour produire un composé β-aminocarbonylé immobilisé sur une résine selon la revendication 1 ou 2, ledit procédé permettant
de faire réagir une imine de formule I: P-Q-N=CH-R immobilisée sur une résine avec un silyléther de formule suivante: 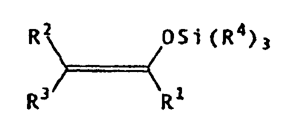 (où R⁴ représente un groupement méthyle)

4. Procédé pour fabriquer un composé β-aminocarbonylé représenté par au moins une des formules (A) et (B), permettant de libérer un composé β-aminocarbonylé immobilisé sur une résine selon la revendication 1 ou 2 à partir de la résine de formule: 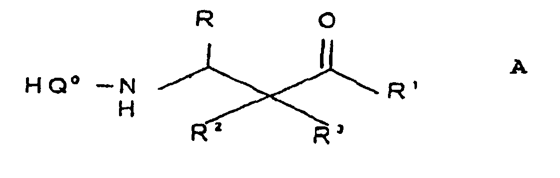 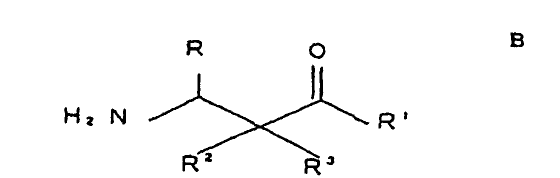 (où Q° représente une partie de la chaîne Q).

5. Procédé pour fabriquer un composé β-aminocarbonylé selon la revendication 4, où le composé β-aminocarbonylé est libéré de la résine par utilisation d'acide de Lewis.
